# EUROPEAN PATENT APPLICATION

(11) **EP 4 389 892 A1**
(43) Date of publication of application: **26.06.2024**
(21) Application number: 22858741.6
(22) Date of filing: 17.08.2022
(51) Int. Cl.: C12N 15/113, A61K 31/7088, A61P 25/16, A61P 25/24, A61P 25/28, A61P 3/10

(54) **ANTISENSE OLIGONUCLEOTIDE TARGETING CAV3.1 GENE AND USES THEREOF**

(30) Priority: 17.08.2021 KR 20210108274
(71) Applicant: Korea Advanced Institute of Science and Technology, Daejeon 34141 (KR)
(72) Inventor: KIM, Dae Soo, Daejeon 34141 (KR); KIM, Jin Kuk, Daejeon 34141 (KR); LEE, Ye Won, Daejeon 34141 (KR); JUNG, Eun Ji, Daejeon 34141 (KR); PARK, Min Sung, Daejeon 34141 (KR); LEE, Sin Jeong, Daejeon 34141 (KR); CHAE, Su Jin, Daejeon 34141 (KR)
(74) Representative: Dehns
(86) International application number: PCT/KR2022/012269
(87) International publication number: WO 2023/022504

(57) **Abstract**

The present invention provides antisense oligonucleotides for the treatment of various T-type calcium channel α1G subunit-related disorders, and more specifically, antisense oligonucleotides for inhibiting expression of the gene encoding Cav3.1, targeting SEQ ID NO: 1, for the treatment of various neuropsychiatric disorders, including Parkinson's disease, epilepsy, essential tremor, depression, anxiety disorders, and unconsciousness.

## Description

### TECHNICAL FIELD

The present invention relates to novel antisense oligonucleotides and uses thereof, and more particularly to antisense oligonucleotides and uses thereof that target the Cav3.1 gene for the treatment of conditions such as Parkinson's disease, essential tremor, epilepsy, depression, and unconsciousness.

### BACKGROUND ART

Parkinson's disease is a disorder of movement caused by the destruction of dopaminergic nerves in the substantia nigra, which is located in the center of the brainstem. Dopamine is an important neurotransmitter that acts on the basal ganglia of the brain and allows us to make precise movements as we wish. Symptoms of Parkinson's disease are evident after 60 to 80 percent of the dopaminergic nerves in the substantia nigra are lost, and pathological examination reveals Lewy bodies caused by the deposition of pathogenic alpha-synuclein protein in various parts of the brain and peripheral nerves. Parkinson's disease is the second most common degenerative brain disorder after Alzheimer's disease, with a prevalence of 1% in people over the age of 60, and an increasing incidence with age.

The core or classic motor characteristics of Parkinson's disease include bradykinesia, tremor, and rigidity, but there are many other motor-related symptoms, including changes in gait and balance, eye movement control, speech and swallowing, and bladder control. In people with Parkinson's disease, bradykinesia manifests as a decrease in movement amplitude, movement speed, and difficulty initiating movements. It can affect the voluntary control of many muscle groups, including eye muscles (resulting in slower onset of saccade movements), speech muscles (leading to softer and sometimes slurred speech), and limb muscles (leading to decreased dexterity). Patients complain of difficulty with fine motor tasks such as button pressing, writing, and using tools, and the tremor is variable. Most, but not all, patients have tremor symptoms (McGregor and Alexandra B. Nelson, Neuron 101: 1042-1056, 2019).

In terms of etiology, Parkinson's disease is inherited in only 5-10% of cases, with the majority of cases being idiopathic. Research on environmental factors in Parkinson's disease has pointed to factors such as exposure to toxins such as 1-methyl-4-phenyl-1,2,3,6-tetrahydropyridine (MPTP), pesticides (rotenone, paraquat), heavy metals (manganese, lead, copper), carbon monoxide, organic solvents, trace metals, and head injury.

The exact cause of the destruction of dopaminergic neurons in the substantia nigra is still unknown.

From the late 1960s to the present day, levodopa has been used to treat Parkinson's disease, which was not only a breakthrough in treatment, but is still recognized as the drug with the greatest anti-Parkinsonian effect. Today, with proper medication, people with Parkinson's disease can maintain a good quality of life for many years and mortality rates have decreased. However, long-term treatment with levodopa is associated with many problems as the disease progresses: approximately 75% of patients experience motor fluctuations, dyskinesias, and other complications after more than six years of levodopa use (Im et al., J. Korean Neurol. Assoc. 19(4): 315-336, 2001).

Recently, the present inventors have shown that optogenetic photostimulation of inhibitory basal ganglia inputs from the globus pallidus of the cerebrum induces action potentials in ventrolateral thalamic neurons and muscle contractions during the inhibition-post period, and these action potentials and muscle contractions are reduced by a reduction in the neuronal population exhibiting post-inhibitory rebound firing by localized knockout of T-type calcium channels in the ventral thalamus, thus linking T-type calcium channels to the pathology of Parkinson's disease (Kim et al., Neuron 95: 1181-1196, 2017).

Voltage-dependent calcium channels are responsible for increasing intracellular calcium concentration in response to neuronal activity (Tsien, R. W., Annu. Rev. Physiol. 45: 341-358, 1983) and are categorized into high-voltage-dependent and low-voltage-dependent channels based on their voltage dependence (Tsien, R. W. et al., Trends Neurosci. 18: 52-54, 1995). T-type calcium channels are typical low-voltage-dependent calcium channels, and in mammals there are three types, Cav3.1 (α1G), Cav3.2 (α1H), and Cav3.3 (α1I), depending on the genotype for the α1 subunit (Perez-Reyes, E., Physiol. Rev. 83: 117-161, 2003). Among them, α1G calcium channels are involved in the generation of burst firing of neurons in the thalamic nucleus and have recently been shown to have important pathological functions (Kim, D. et al., Science 302: 117-119, 2003; Kim, D. et al., Neuron 31: 35-45, 2001). Pathologies associated with α1G calcium channels include abdominal pain (Korean Patent No. 868735), epilepsy (Korean Patent No. 534556), anxiety disorders (Korean Patent No. 958291), Dravet syndrome (Korean Public Patent Publication No. 10-2019-0139302), frontal lobe dysfunction following irreversible brain injury (Korean Public Patent Publication No. 10-2013-0030011), neuropathic pain (Choi et al., Proc. Natl. Acad. Sci. U.S.A., 113(8): 2270-2275, 2016; WO2006064981A1), Angelman syndrome and/or Prader-Willi syndrome (WO2017070680A1), depression (CN108853510A), rheumatoid arthritis (WO2020203610A1), concentration disorders (Korean Patent No. 1625575), diabetes (CN10264172A), and essential tremor (Korean Patent No. 958286). Interestingly, transgenic mice in which the Cav3.1 T-type calcium channel is knocked out (Cav3. I^{-/-}) are not lethal and ostensibly develop normally.

Drugs that block the function of T-type calcium channels are needed to ameliorate these pathologies, but there are several problems. First, it is difficult for various T-type calcium channel blockers, including etosuximide, to selectively inhibit only Cav3.1 due to the high homology between the three types of T-type calcium channels described above. Second, etosuximide, which is used clinically under the brand name Zarontin^{®}, also inhibits voltage-dependent Na⁺ and K⁺ channels, which are important for generating nerve signals (Leresche et al., J. Neurosci. 18(13): 4842-4853, 1998), with known side effects including headache, dizziness, and vomiting. Third, the structural similarity of calcium channels leads to cross-talk between calcium channel inhibitors and other types of calcium channels, such as N-type and P/Q-type channels, which are important for neurotransmitter release in all nerves and, when blocked, cause significant disruption of brain function and life. Fourth, even blockers that are selective for Cav3.1 channels may interfere with the function of Cav3.1 expressed in cardiac nerves, leading to abnormalities in cardiac function (Mangoni et al., Circ. Res. 98: 1422-1430, 2006).

For this reason, nucleic acid molecule-based drugs such as siRNA and shRNA that target only the Cav3.1 gene have been developed, but they have major problems in actual clinical application, such as the need to be introduced into a separate vector such as a lentivirus and the need to be injected locally and precisely into the required brain area using a highly invasive device such as a stereotaxic injector.

Therefore, there is an urgent need to develop a new class of drugs that selectively inhibit only α1G T-type calcium channels (Cav3.1) without other side effects.

### DETAILED DESCRIPTION OF THE INVENTION

### TECHNICAL PROBLEM

Accordingly, the present invention is directed to solving the problems described above, and an object of the present invention is to provide a drug that more efficiently inhibits the expression of α1G T-type calcium channels, thereby providing a therapeutic agent for inhibition of α1G T-type calcium channels, such as in Parkinson's disease. However, these objects are exemplary and do not limit the scope of the present invention.

### TECHNICAL SOLUTION

In an aspect of the present invention, there is provided an antisense oligonucleotide targeting the nucleic acid sequence represented by SEQ ID NO: 1 for inhibiting Cav3.1 gene expression

In another aspect of the present invention, there is provided a pharmaceutical composition for treating Parkinson's disease comprising the antisense oligonucleotide as an active ingredient.

In another aspect of the present invention, there is provided a pharmaceutical composition for treating depression comprising the antisense oligonucleotide as an active ingredient.

In another aspect of the present invention, there is provided a pharmaceutical composition for treating essential tremor comprising the antisense oligonucleotide as an active ingredient.

In another aspect of the present invention, there is provided a pharmaceutical composition for treating epilepsy comprising the antisense oligonucleotide as an active ingredient.

In another aspect of the present invention, there is provided a pharmaceutical composition for treating anxiety disorders comprising the antisense oligonucleotide as an active ingredient.

In another aspect of the present invention, there is provided a pharmaceutical composition for recovering consciousness in a subject in an unconscious state comprising the antisense oligonucleotide as an active ingredient.

In another aspect of the present invention, there is provided a method of treating a subject suffering from Parkinson's disease, comprising administering a therapeutically effective amount of the antisense oligonucleotide to the subject.

In another aspect of the present invention, there is provided a method of treating a subject suffering from depression, comprising administering a therapeutically effective amount of the antisense oligonucleotide to the subject.

In another aspect of the present invention, there is provided a method of treating a subject suffering from essential tremor, comprising administering a therapeutically effective amount of the antisense oligonucleotide to the subject.

In another aspect of the present invention, there is provided a method of treating a subject suffering from epilepsy comprising administering a therapeutically effective amount of the antisense oligonucleotide to the subject.

In another aspect of the present invention, there is provided a method of treating a subject suffering from an anxiety disorder comprising administering a therapeutically effective amount of the antisense oligonucleotide to the subject having an anxiety disorder.

In another aspect of the present invention, there is provided a method of recovering consciousness in a subject in an unconscious state comprising administering a therapeutically effective amount of the antisense oligonucleotide to the subject.

### ADVANTAGEOUS EFFECTS

Unlike conventional T-type channel blockers, antisense oligonucleotides according to an embodiment of the present invention not only specifically inhibit the expression of the Cav3.1 gene, but also have few side effects when administered *in vivo*, making them highly effective in treating a variety of conditions associated with Cav3.1 activity, such as Parkinson's disease, neuropathic pain, attention and learning problem, and anxiety disorders. However, the effects of the present invention are not limited thereto.

### DESCRIPTION OF THE DRAWINGS

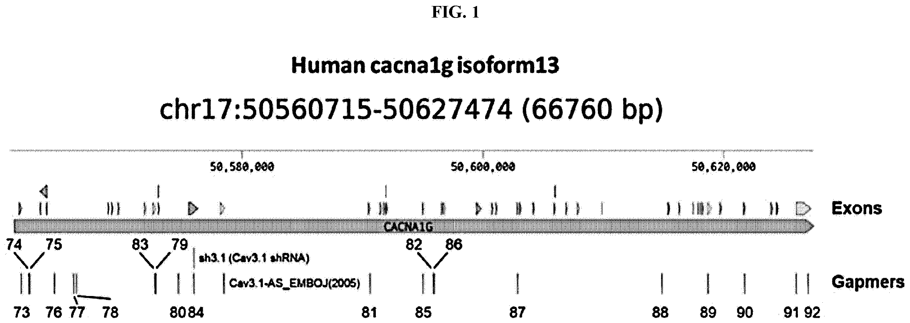

FIG. 1 is a genomic map showing the genomic DNA structure of isoform 13 of the CACNA1G gene, the target of the antisense oligonucleotides of the present invention, and the target locations of candidate antisense oligonucleotides designed to target the CACNA1G gene.

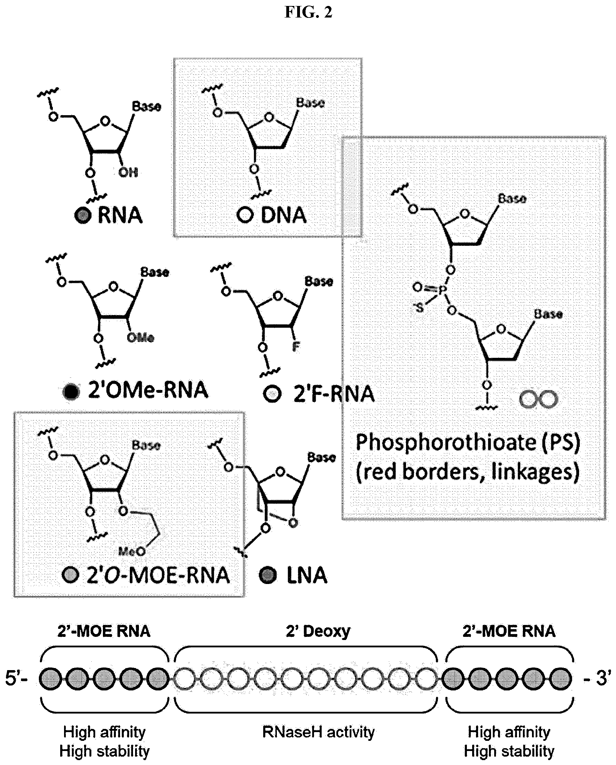

FIG. 2 is a schematic illustration of the structure of a gapmer, an antisense oligonucleotide prepared in accordance with one embodiment of the present invention, and the types of modifications used.

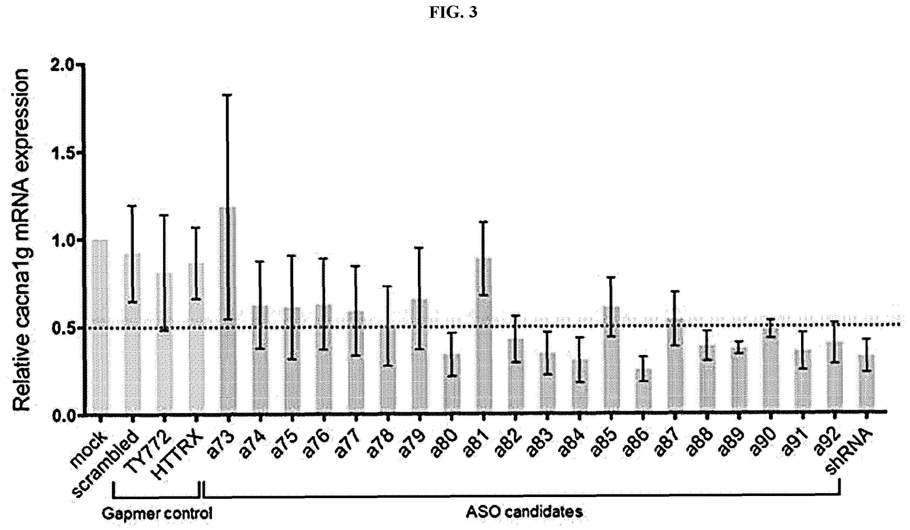

FIG. 3 is a graph showing the knockdown efficiency of the CACNA1G gene when cells are transfected with candidate antisense oligonucleotides according to one embodiment of the present invention.

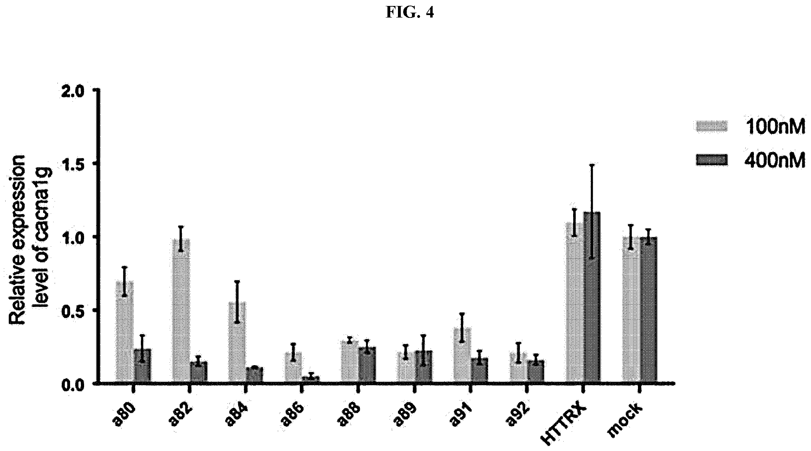

FIG. 4 is a graph representing the results of comparing the degree of inhibition of expression of the CACNA1G gene according to treated concentration when cells are treated with antisense oligonucleotide candidates according to one embodiment of the present invention.

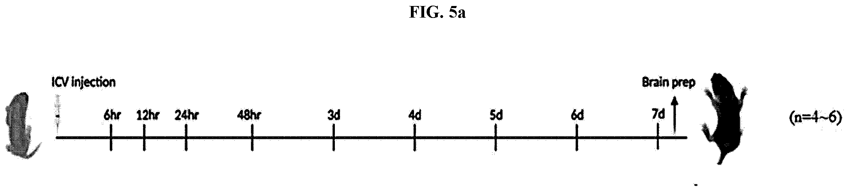

FIG. 5a is a schematic representation of an experimental schedule for analyzing the toxicity of antisense oligonucleotides according to one embodiment of the present invention and their ability to regulate the expression of the CACNA1G gene *in vivo*;

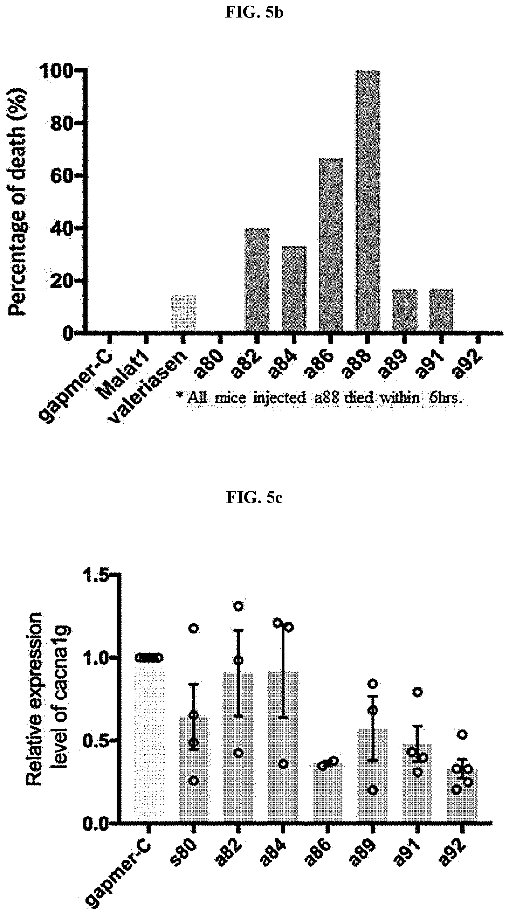

FIG. 5b is a graph depicting the survival rate up to 7 days after administration of eight antisense oligonucleotide candidates according to one embodiment of the present invention to experimental animals; and FIG. 5c is a graph depicting the expression of the CACNA1G gene in brains excised from the experimental animals as measured by qRT-PCR.

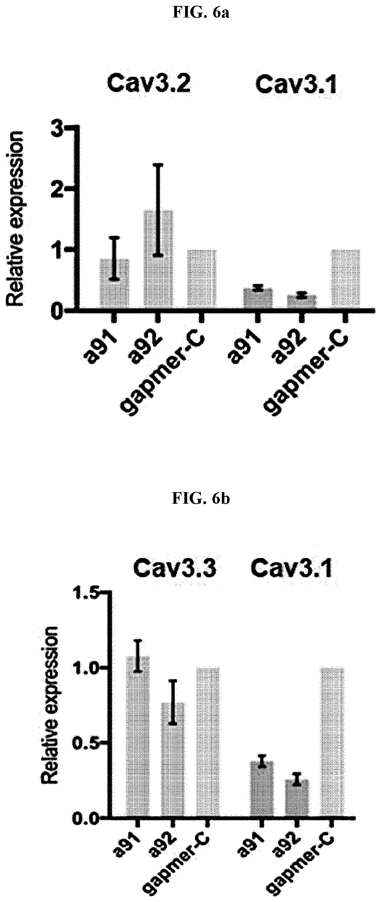

FIG. 6a is a graph showing the results of a qRT-PCR assay performed to measure the cross-reactivity of antisense oligonucleotides according to one embodiment of the present invention against Cav3.2; and FIG. 6b is a graph showing the results of a qRT-PCR assay performed to measure the cross-reactivity of an antisense oligonucleotides according to one embodiment of the present invention against Cav3.3.

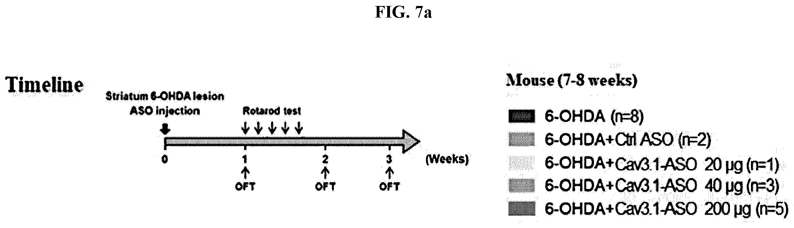

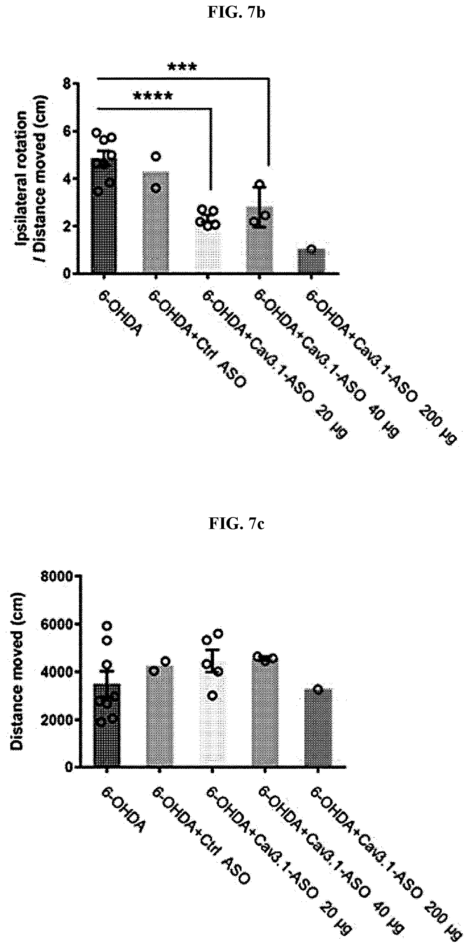

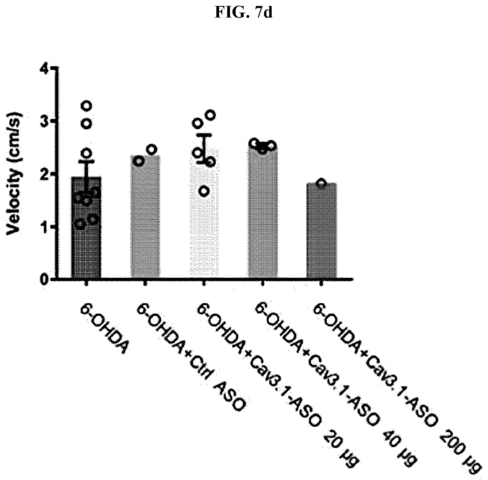

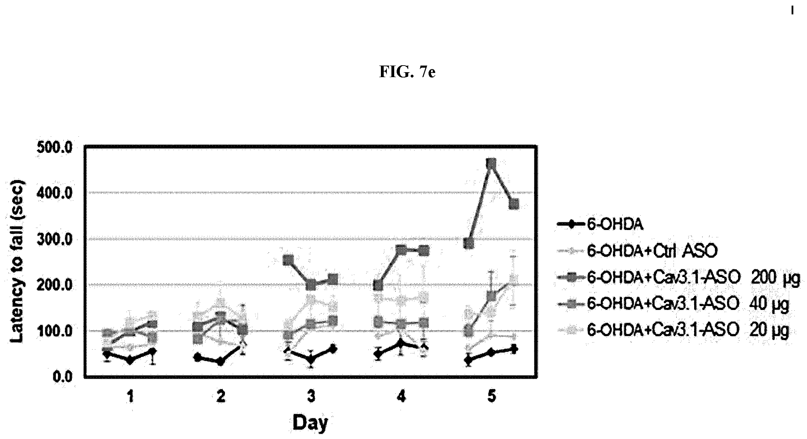

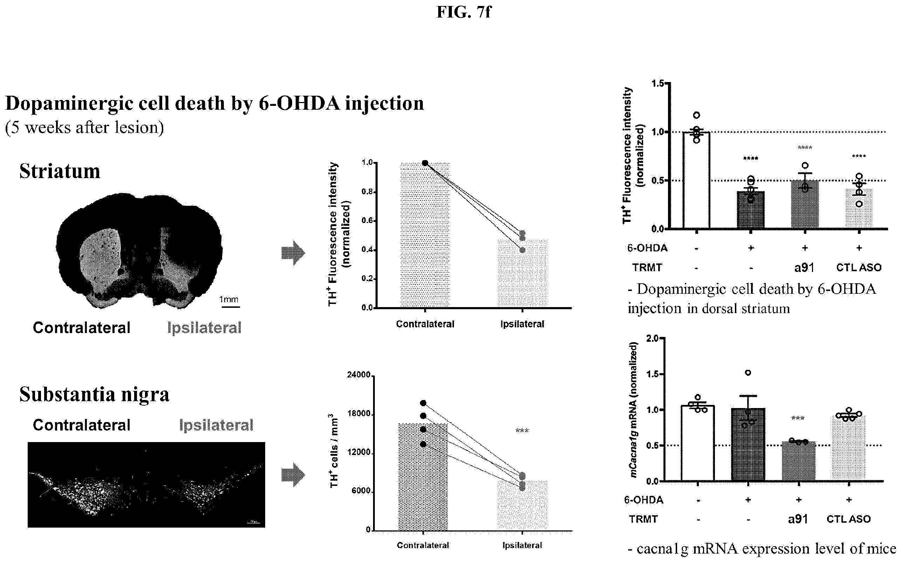

FIG. 7a is a schematic diagram representing an animal experiment schedule to test the therapeutic effect of an antisense oligonucleotide according to one embodiment of the present invention on Parkinson's disease; FIG. 7b is a graph representing the results of a lateral rotation travel distance measurement in an open field test performed one week after administration of an antisense oligonucleotide according to one embodiment of the present invention; and FIG. 7c is a graph representing the results of a travel distance measurement in the open field test; FIG. 7d is a graph showing the results of measuring the speed of locomotion in the open field test; FIG. 7e is a graph showing the results of measuring the time to fall, measured by performing an accelerated rotarod test for 5 days at 1-day intervals, after 1 week of administration of an antisense oligonucleotide according to one embodiment of the present invention in the above animals; and FIG. 7f is a series of figures showing the results of histochemical analyses performed to determine the mechanism of action of an antisense oligonucleotide according to one embodiment of the present invention.

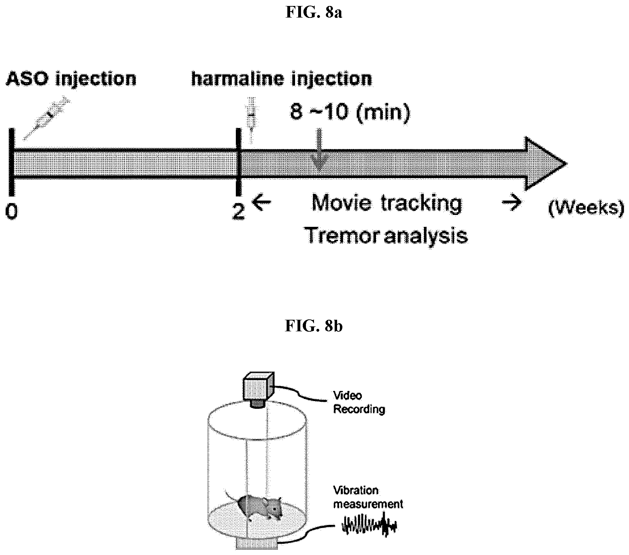

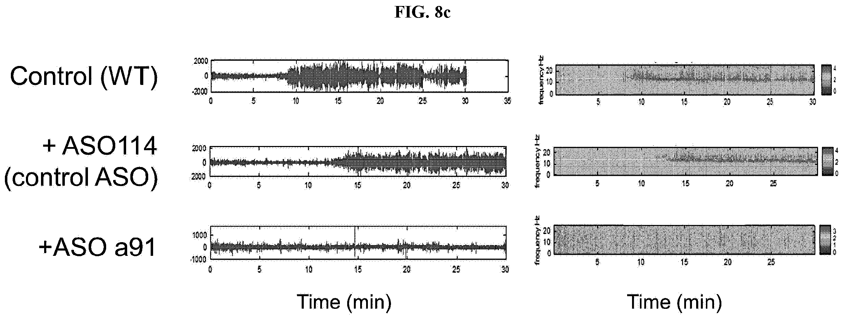

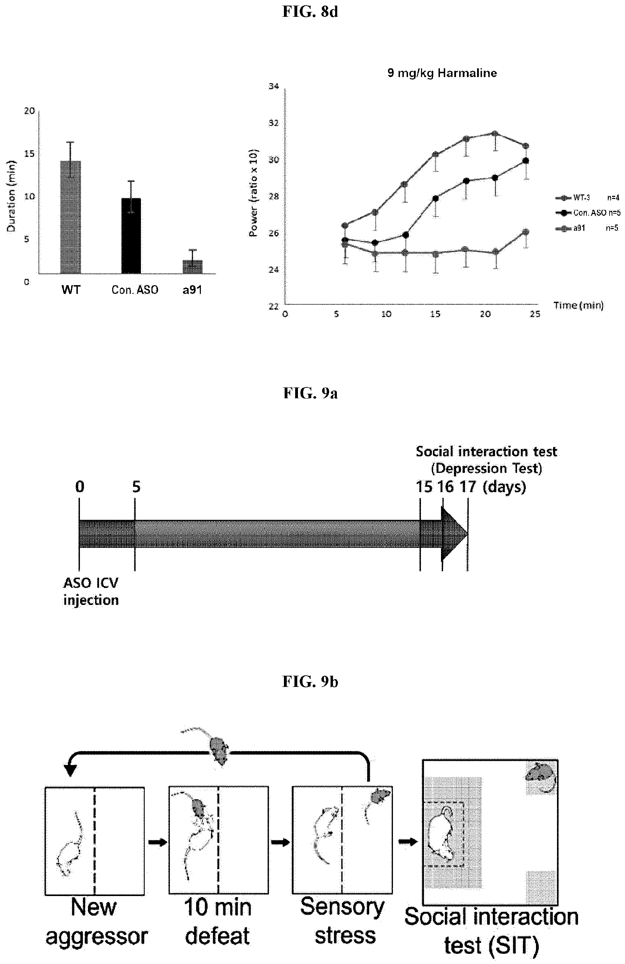

FIG. 8a is a schematic diagram illustrating a schedule of animal experiments to test the effectiveness of an antisense oligonucleotide in treating essential tremor according to one embodiment of the present invention; and FIG. 8b is a schematic diagram illustrating an apparatus for measuring tremor symptoms caused by essential tremor; FIG. 8c is a schematic diagram representing the results of measuring the severity of tremor in the form of a spectrogram upon administration of an antisense oligonucleotide according to one embodiment of the present invention; and FIG. 8d is a series of graphs quantifying the results of FIG. 8c, wherein the left panel is a measurement of the duration of the tremor and the right panel is a quantification of the intensity of the tremor over time.

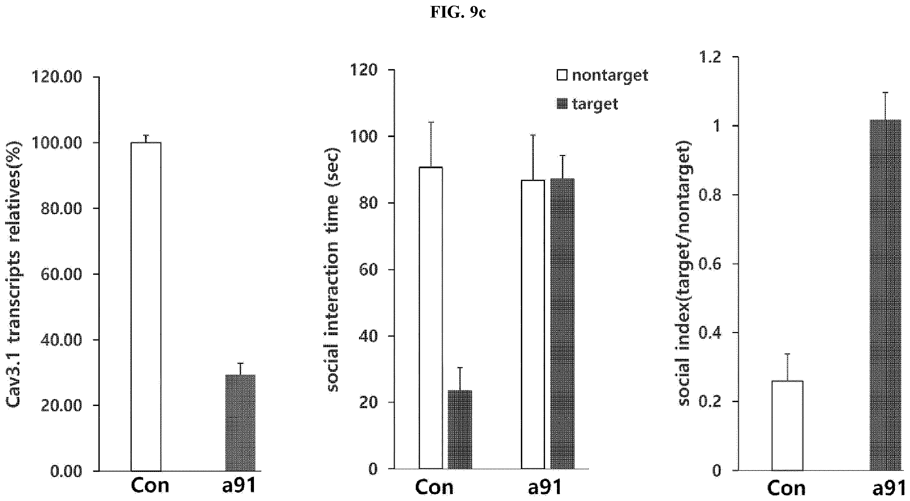

FIG. 9a is a schematic diagram illustrating a schedule for a social stress-induced depression animal experiment to test the therapeutic effect of an antisense oligonucleotide according to one embodiment of the present invention on chronic sustained stress-induced depression; FIG. 9b is an overview diagram schematically depicting a method of testing for chronic social defeat stress-induced depression performed during a 10-day course of social stress from day 5 to day 17 after administration of an antisense oligonucleotide according to one embodiment of the present invention; FIG. 9c is a series of graphs representing quantification of a measure of chronic depression upon administration of an antisense oligonucleotide according to one embodiment of the present invention, wherein the left panel depicts Cav3. 1 mRNA expression quantified by qRT-PCR, the central panel depicts a measure of social interaction time, and the right panel represents a measure of sociability index.

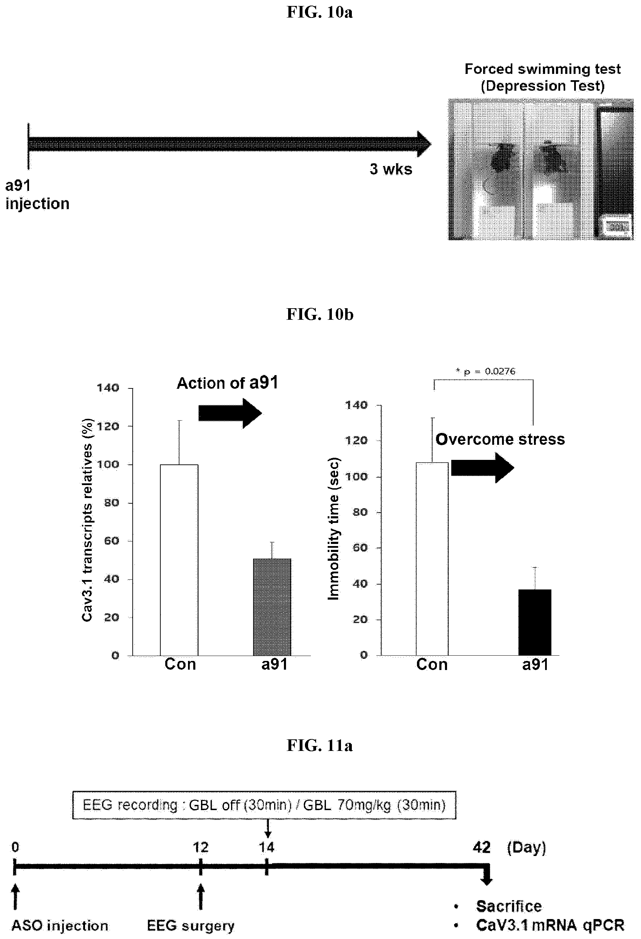

FIG. 10a is a schematic diagram illustrating a schedule of forced swimming animal test to verify the therapeutic effect of an antisense oligonucleotide according to one embodiment of the present invention on short-term acute stress-induced depression; and FIG. 10b is a graph showing the quantification of the forced swimming-induced acute depression scale upon administration of an antisense oligonucleotide according to one embodiment of the present invention, wherein the left panel shows the Cav3. 1 mRNA expression quantified by qRT-PCR, and the right panel shows the results of measuring immobility time, which is a measure of stress.

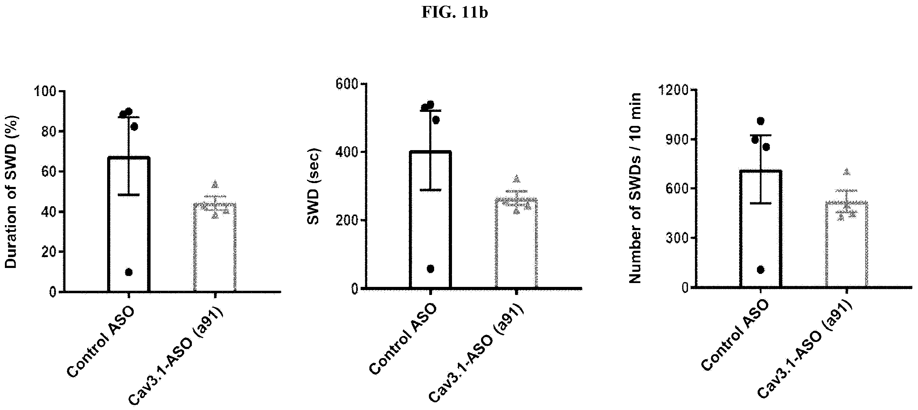

FIG. 11a is a schematic diagram illustrating an animal experimental schedule to verify the therapeutic effect of antisense oligonucleotides on epilepsy according to one embodiment of the present invention, in which electroencephalography (EEG) probe insertion is performed 12 days after antisense oligonucleotide administration, and EEG is measured for 30 minutes before and after treatment with GBL (70 mg/kg), which induces absence seizures on day 14; FIG. 11b is a series of graphs showing EEG measurements quantifying the extent of reduction in spike and wave discharges (SWDs) in seizure-specific EEG wavebands upon administration of an antisense oligonucleotide according to one embodiment of the present invention in the above animals, wherein the left panel represents the duration of SWDs, the total time of SWDs, and the right panel represents the number of SWDs.

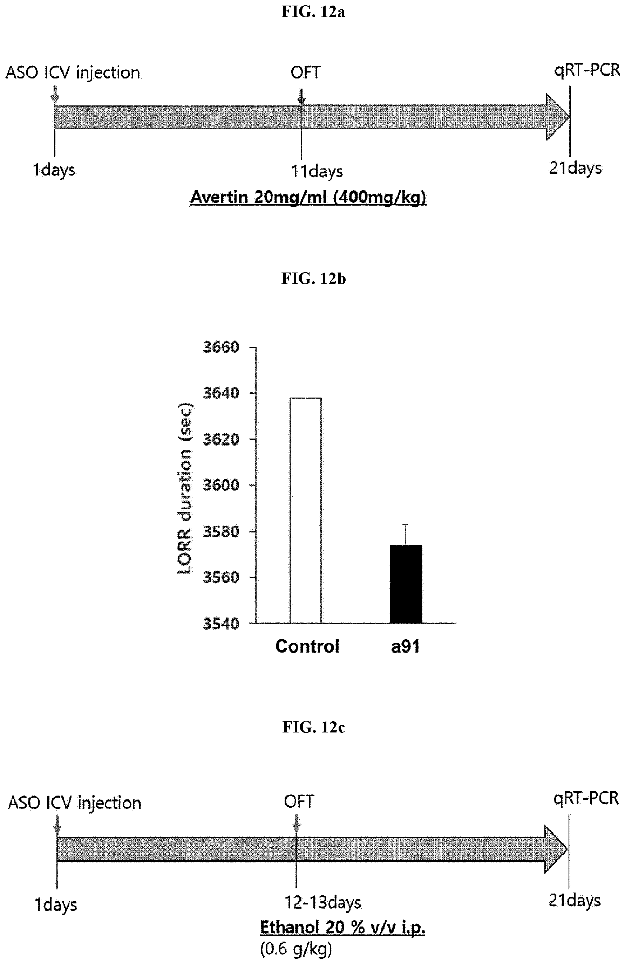

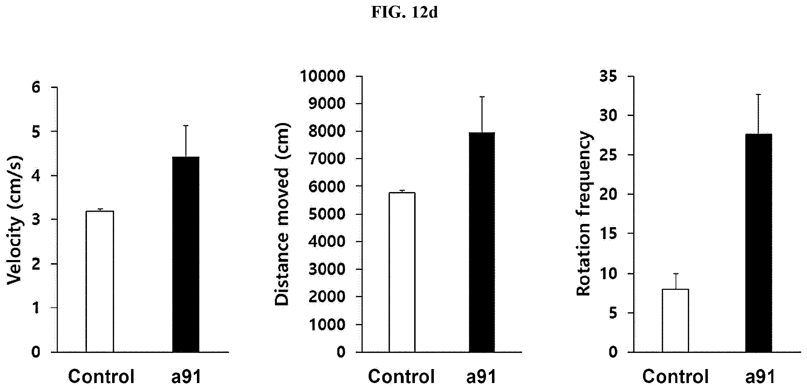

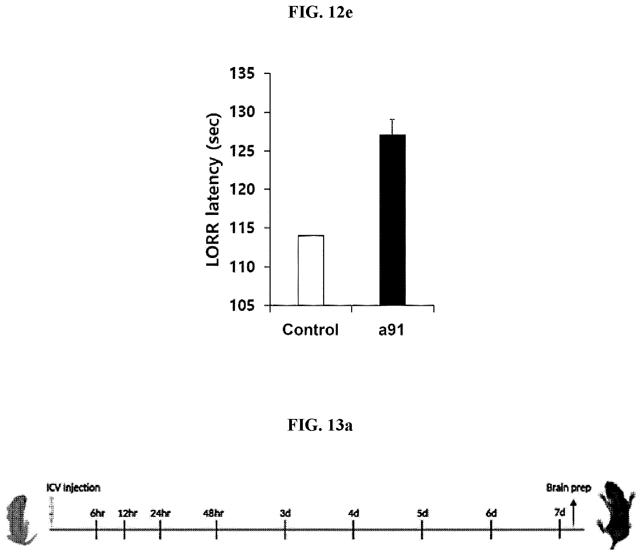

FIG. 12a is a schematic diagram illustrating an animal experiment schedule to test the therapeutic effect of an antisense oligonucleotide according to one embodiment of the present invention on the anesthetic, arbutin-induced loss of consciousness; and FIG. 12b is a schematic diagram showing the time to recovery from arbutin-induced loss of consciousness upon administration of an antisense oligonucleotide according to one embodiment of the present invention; FIG. 12c is a graph representing the results of validating the effectiveness of the treatment of loss of consciousness by measuring the time to recover from a loss of consciousness (loss of righting reflex, LORR); and FIG. 12d is a schematic diagram illustrating another schedule of animal test to validate the awakening effect of an antisense oligonucleotide according to one embodiment of the present invention after ethanol treatment; FIG. 12d is a series of graphs representing the results of verifying therapeutic effect on unconsciousness by measuring the degree of awakening caused by low concentrations of ethanol when administering an antisense oligonucleotide according to one embodiment of the present invention, wherein the left panel shows the result of measuring the speed of the experimental animal, the central panel shows the result of measuring the distance traveled by the experimental animal, and the right panel represents the result of measuring the frequency of rotation of the experimental animal; and FIG. 12e is a graph showing the time to loss of consciousness (LORR latency) after treating a high concentration (3. 5 g/kg) of ethanol.

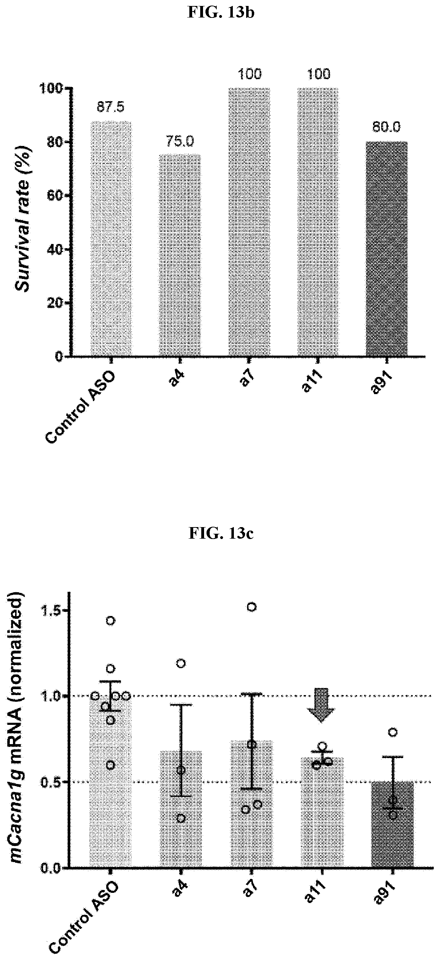

FIG. 13a is a schematic diagram representing a schedule of animal experiments using an antisense oligonucleotide targeted to the vicinity of the target sequence of antisense oligonucleotide A91 according to one embodiment of the present invention; FIG. 13b is a graph showing the survival rate of experimental animals up to 7 days after administration of various antisense oligonucleotides according to one embodiment of the present invention; and FIG. 13c is a graph showing the results of measuring the expression of Cav3. 1 in brains excised from experimental animals sacrificed after 7 days of treatment with various antisense oligonucleotides according to one embodiment of the invention.

### BEST MODE

### Definition of terms:

As used herein, the term "oligonucleotide" refers to a polynucleotide formed from natural nucleobase and pentafuranosyl group (sugar) forming nucleosides that are linked together by natural phosphodiester linkages. Thus, the term "oligonucleotide" refers to natural species or synthetic species formed from natural subunits or close homologs thereof.

As used herein, the term "oligonucleotide" is a concept that includes any nucleotide, preferably deoxyribonucleotide (DNA), ribonucleotide (RNA), or DNA/RNA hybrid nucleic acid molecule that is a mixture of DNA and RNA. The term refers only to the primary structure of the molecule; therefore, the term includes double-stranded or single-stranded DNA and double-stranded or single-stranded RNA.

In addition, the term "oligonucleotide" includes a segment that is artificial, rather than natural, and performs a function similar to that of a natural oligonucleotide. An oligonucleotide can have a sugar portion, a nucleobase portion, or a modified inter-nucleotide linkage. Among the possible modifications, preferred modifications are 2'-O-alkyl derivatives of the sugar portion, in particular 2'-O-ethyloxymethyl or 2'-O-methyl, and/or phosphorothioate, phosphorodithioate or methylphosphonate for the inter-nucleotide backbone.

As used herein, the term "inter-nucleoside linker" refers to a group capable of covalently linking two nucleosides, such as between DNA units, between DNA units and nucleotide analogs, between two non-LNA units, between a non-LNA unit and an LNA unit, or between two LNA units. In naturally occurring nucleic acid molecules, phosphodiester is used as such an inter-nucleoside linker, but such phosphodiester can be substituted with similar linkers such as phosphorothioate as described above. Such a linker may be any one or more selected from the group consisting -O-P(O)₂-O-, -O-P(O,S)-O-, -O-P(S₎₂-O-, -S-P(O)₂-O-, -S-P(O,S)-O-, -S-P(S)₂-O-, -O-P(O)₂-S-, -O-P(O,S)-S-, -S-P(O)₂-S-, -O-PO(R)-O-, O-PO(OCH₃)-O-, -O-PO(NR)-O-, -O-PO(OCH₂CH₂S-R)-O-, -O-PO(BH₃)-O-, -O-PO(NR)-O-, -O-P(O)₂-NR-, -NR-P(O)₂-O-, -NR-CO-O-, -NR-CO-NR-, -O-CO-O-, -O-CO-NR-, -NR-CO-CH2-, -O-CH₂-CO-NR-, -O-CH₂-CH₂-NR-, -CO-NR-CH₂-, -CH₂-NR-CO-, -OCH₂-CH₂-S-, -S-CH₂-CH₂-O-, -S-CH₂-CH₂-S-, -CH₂-SO₂-CH₂-, -CH₂-CO-NR-, -O-CH₂-CH₂-NR-CO-, -CH₂-NCH₃-O-CH₂-, wherein R is a hydrogen or an alkyl group having 1 to 4 carbons.

As used herein, the term "targeted" means capable of hybridizing complementarily to a specific nucleic acid sequence.

As used herein, the terms "hybridizable," or "hybridization" are generally used to mean the formation of hydrogen bonds, also known as Watson-Crick bonds between complementary bases, on two strands of nucleic acid to form a double helix duplex or triplex (if the oligonucleotide is double-stranded).

As used herein, the term "gene encoding Cav3.1" refers to the genomic sequence of cacna1g, which includes the introns and exons of the gene encoding the α1G subtype of T-type calcium channel. The cacnalg gene is known to be located on chromosome 17 in humans and to have a size of 66,760 bp based on NCBI Reference Sequence: NC_000017.11.

As used herein, the term "product of the gene encoding Cav3.1" refers to the mRNA sequence produced by transcription from the cacnalg genomic sequence. Said mRNA may comprise both pre-spliced and post-spliced transcripts.

The antisense oligonucleotide according to one embodiment of the invention preferably hybridizes specifically to a gene encoding Cav3.1 or a product thereof. Antisense oligonucleotides according to the present invention can hybridize to DNA of genes encoding Cav3.1 and/or mRNA derived from the gene. In particular, an antisense oligonucleotide according to one embodiment of the present invention can target the nucleotide sequence described by SEQ ID NO: 1, which includes intron 37 and exon 38 regions of the mRNA encoding Cav3.1. In other words, an antisense oligonucleotide according to one embodiment of the present invention can inhibit the expression of Cav3.1 by hybridizing to both pre- and post-spliced mRNA encoding Cav3.1, as long as the location is appropriate. Antisense oligonucleotides according to one embodiment of the present invention comprise nucleotides that are sufficiently identical to hybridize specifically.

As used herein, the term "specific hybridization" means that there is a sufficient degree of complementarity to avoid non-specific hybridization of the oligonucleotide to non-targeted sequences, particularly under conditions requiring specific hybridization. The oligonucleotide need not be 100% complementary to the target nucleic acid sequence to be specifically hybridized. In particular, an oligonucleotide having a degree of complementarity of at least about 80% can specifically hybridize to a nucleic acid selected as a target.

As used herein, the terms "LNA unit", or "LNA monomer", "LNA residue" "locking nucleic acid unit", "locking nucleic acid monomer" or "locking nucleic acid residue" refer to a bicyclic nucleoside analog. LNA units are described in detail in WO1999014226A, WO2000056746A, WO2000056748A, WO200125248A, WO2002028875A, WO2003006475A and WO2003095467A. An LNA unit may also be defined by its chemical formula. Therefore, as used herein, "LNA unit" means a compound whose formula is represented by the following structural formula: 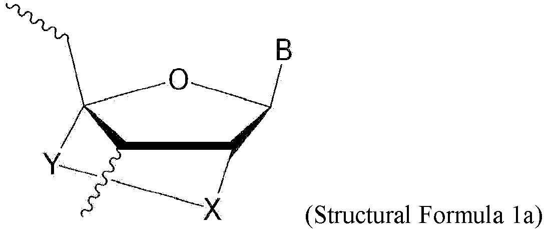 or

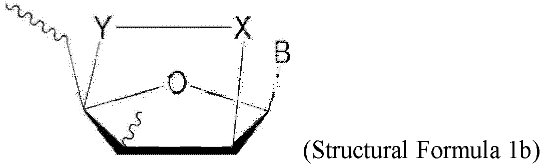

(In the above structural formula, X is selected from the group consisting of O, S and NR, R is H or an alkyl group having 1 to 4 carbons, and Y is CH₂).

In the above structural formulas 1a and 1b, when X is S, it is referred to as a "thio-LNA unit", when X is NR, it is referred to as an "amino-LNA unit", and when X is O, it is referred to as an "oxy-LNA unit".

As used herein, the term "gapmer antisense oligonucleotide" refers to a short single-stranded antisense oligonucleotide comprising a central DNA sequence between the LNA sequences at either end that is taken up into the cell without a transfection agent and interferes with target mRNA expression by induction of RNase H activation by a process referred to as gymnosis (Hvam et al., Mol. Ther. 25: 1710-1717, 2017). Thus, it is a kind of hybrid nucleic acid molecule in which LNA, a kind of an RNA and a DNA are linked to each other. The LNA at both ends makes it resistant to nucleases and has a high affinity for the target sequence, while the DNA sequence inside acts as an RNase H activation site to induce RNA-specific degradation in the DNA/RNA hybrid. Typically, the LNA at the sock end has a length of 3 to 5 nt and the DNA in the center has a length of about 10 nt. However, in one embodiment of the present invention, the RNA at both ends of the antisense oligonucleotide may use thymine (T) instead of uracil (U) as a base.

### Detailed Description of the Invention:

In an aspect of the present invention, there is provided an antisense oligonucleotide targeting the nucleic acid sequence represented by SEQ ID NO: 1 for inhibiting the expression of a gene encoding Cav3.1.

The antisense oligonucleotide is a nucleic acid molecule capable of hybridizing to the nucleic acid sequence represented by SEQ ID NO: 1 and may have a length of, for example, 11 to 30 nt or 12 to 29 nt. In a preferred embodiment, an antisense oligonucleotide of the present invention may have a length of 13 to 28 nt, more preferably a length of 14 to 27 nt, 15 to 26 nt, 16 to 25 nt, 17 to 24 nt, 18 to 23 nt, or 19 to 22 nt. More specifically, an antisense oligonucleotide according to one embodiment of the present invention can have a length of 11, 12, 13, 14, 15, 16, 17, 18, 19, 20, 21, 22, 23, 24, 25, 26, 27, 28, 29, or 30 nt.

The antisense oligonucleotide according to one embodiment of the present invention may be a nucleic acid molecule having a length of 7 to 30 nt. The nucleic acid sequence of SEQ ID NO: 1 comprises exon 38 of the gene encoding Cav3.1 and a 3'-terminal portion of intron 37 adjacent thereto. Preferably, the antisense oligonucleotide according to one embodiment of the present invention may comprise the nucleic acid sequence designated by SEQ ID NO: 20 or SEQ ID NO: 21.

The antisense oligonucleotide may be a DNA, RNA or DNA/RNA hybrid nucleic acid molecule, wherein some or all of the phosphodiester (PO) backbone is substituted with another form, for example phosphorothioate (PS), phosphorodithioate or methylphosphonate, some or all of the 2'-OH of the sugar portion may be modified, examples of such 2'-OH modifications include nucleotide analogs substituted with -O-CH₃ (-OMe), -O-CH₂-CH₃ (-OEt), -O-CH₂-CH₂-O-CH₃ (-MOE), -O-CH₂-CH₂-CH₂-NH₂, -O-CH₂-CH₂-CH₂-OH, -OF or -F.

Optionally, the antisense oligonucleotide according to one embodiment of the invention may comprise a nucleotide analog in which the sugar portion is modified, for example, a so-called locked nucleic acid (LNA) unit in which an additional ring is formed on the sugar portion. Such LNAs are described above. At least about 30% of the total nucleotides may be nucleotide analogs, for example at least about 33%, for example at least about 40%, for example at least about 50%, for example at least about 60%, for example at least about 66%, for example as at least about 70%, for example at least about 80%, or for example at least about 90%. The antisense oligonucleotide according to one embodiment of the present invention may comprise a nucleotide sequence comprising only nucleotide analog sequences.

Modification of the backbone and sugar portions of such oligonucleotides is intended to improve drug stability and pharmacokinetic properties by increasing the stability of hybridization with the target sequence or by inhibiting a significant decrease in the half-life in the body due to degradation by nucleases and the like.

Preferred modifications of the invention include chimeric oligonucleotides. The oligonucleotides comprise at least two chemically different regions, each comprising one or more nucleotides. In particular, they comprise one or more regions containing modified nucleotides conferring one or more favorable properties, such as better biological stability, increased bioavailability, increased cellular internalization, or increased affinity for a target RNA.

The degree of complementarity between two sequences of nucleic acids of the same length is determined by aligning the two sequences and comparing the sequences that are complementary to the first and second sequences. The degree of complementarity is calculated by determining the number of identical positions between the two sequences whose nucleotides are compared, dividing the number of identical positions by the total number of positions, and multiplying the result obtained by 100 to obtain the degree of complementarity between these two sequences.

The antisense oligonucleotide according to one embodiment of the present invention may be a gapmer, wherein said gapmer is a nucleic acid molecule comprising a 3-5 nt long RNA unit at each end and 8-12 nt long DNA spaced between the RNA units and LNA. In this case, said RNA unit may comprise at least one LNA.

In another aspect of the present invention, there is provided a pharmaceutical composition for treating Parkinson's disease comprising the antisense oligonucleotide as an active ingredient.

The association of Parkinson's disease with Cav3.1 is well described in the prior art (Park et al., Front. Neural Circuits, 7: 172, 2013; Rubit et al., Eur. J. Neurosci: 36(2): 2213-2228, 2012; Xiang et al., ASC Chem. Neurosci. 2(12): 730-742, 2012). Furthermore, the present inventors experimentally demonstrated that antisense oligonucleotides according to one embodiment of the present invention, which specifically bind to Cav3.1, particularly to the region including intron 37-exon 38, to inhibit the expression of Cav3.1, but without other side effects, can significantly alleviate abnormal muscle symptoms in Parkinson's disease model animals. Thus, antisense oligonucleotides according to one embodiment of the present invention, which can specifically inhibit the expression of Cav3.1, may be a highly efficient therapeutic agent in the treatment of Parkinson's disease.

In another aspect of the present invention, there is provided a pharmaceutical composition for treating depression comprising the antisense oligonucleotide as an active ingredient.

Recent studies have reported that T-type calcium channels correlate with anxiety-related behaviors. Specifically, it has been reported that opening of T-type calcium channels induces anxiety-related behaviors, and conversely, administration of T-type calcium channel blockers reduces anxiety symptoms (Kaur et al., J. Basic Clin. Physiol. Pharmacol., 31(3): 20190067, 2020). Moreover, the present inventors have experimentally confirmed that depression-related symptoms are alleviated in transgenic mice in which the Cav3.1 gene is knocked out. Thus, antisense oligonucleotides according to one embodiment of the present invention targeting the Cav3.1 gene can be used to treat depression. Furthermore, the inventors have demonstrated in animal studies that the antisense oligonucleotides in one embodiment of the present invention are effective in the treatment of depression.

According to one aspect of the present invention, there is provided a pharmaceutical composition for treating essential tremor comprising said antisense oligonucleotide as an active ingredient.

The present inventors have shown that essential tremor can be treated by specifically inhibiting Cav3.1 (α1G subunit of T-type calcium channel) (Korean Patent No. 958286). Therefore, an antisense oligonucleotide capable of specifically inhibiting the expression of Cav3.1 according to one embodiment of the present invention can be used to treat essential tremor. In addition, the inventors have demonstrated in animal studies that the antisense oligonucleotides in accordance with one embodiment of the present invention can be used for the treatment of essential tremor.

In another aspect of the present invention, there is provided a pharmaceutical composition for treating epilepsy comprising the antisense oligonucleotide as an active ingredient.

It is known that T-type calcium channel blockers, which specifically inhibit T-type calcium channels, reduce thalamic firing and thereby suppress seizure symptoms (Powell et al., Br. J. Clin. Pharmacol., 77: 729-739, 2014; Casillas-Espinosa et al., PLoS One, 10: 30130012, 2015). Thus, the antisense oligonucleotides according to one embodiment of the present invention can be used for the treatment of epilepsy. Furthermore, the inventors have demonstrated in animal studies that the antisense oligonucleotides according to one embodiment of the present invention can be used for the treatment of epilepsy.

In the pharmaceutical composition, the epilepsy may be an absence seizure.

In another aspect of the present invention, there is provided a pharmaceutical composition for treating anxiety disorders comprising the antisense oligonucleotide as an active ingredient.

The present inventors have shown that administration of mibefradil, a T-type calcium channel selective blocker, to mice with a knocked-out gene for phospholipaseβ4 (PLCβ4), a model animal for anxiety disorders, normalizes the memory extinction ability that was impaired in said mice, whereas, administration of mibefradil and efonidipine to the MD of normal mice rapidly extinguishes fear memories in said mice and reduces the recall ability of said extinguished memories (Korean Patent No. 958291). Thus, the antisense oligonucleotides capable of specifically inhibiting the expression of Cav3.1 according to one embodiment of the present invention may be used for the treatment of anxiety disorders.

In another aspect of the present invention, there is provided a pharmaceutical composition for recovering consciousness in a subject in unconscious state comprising the antisense oligonucleotide as an active ingredient. The present inventors experimentally demonstrated that administration of the antisense oligonucleotides in one embodiment of the present invention to experimental animals in which unconsciousness has been induced promotes recovery of consciousness. Thus, the antisense oligonucleotide according to one embodiment of the present invention can be used as a drug to restore consciousness in unconscious patients, including comatose patients.

In another aspect of the present invention, there is provided a method of treating a subject suffering from Parkinson's disease, comprising administering a therapeutically effective amount of the antisense oligonucleotide to the subject.

In another aspect of the present invention, there is provided a method of treating a subject suffering from depression comprising administering a therapeutically effective amount of the antisense oligonucleotide to the subject.

In another aspect of the present invention, there is provided a method of treating a subject suffering from essential tremor, comprising administering a therapeutically effective amount of the antisense oligonucleotide to the subject.

In another aspect of the present invention, there is provided a method of treating a subject suffering from epilepsy comprising administering a therapeutically effective amount of the antisense oligonucleotide to the subject.

In said method, said epilepsy may be an absence seizure.

In another aspect of the present invention, there is provided a method of treating a subject suffering an anxiety disorder comprising administering a therapeutically effective amount of the antisense oligonucleotide to the subject.

In another aspect of the present invention, there is provided a method of recovering consciousness in a subject in an unconscious state comprising administering a therapeutically effective amount of the antisense oligonucleotide to the subject.

The composition may comprise a pharmaceutically acceptable carrier, and may further comprise pharmaceutically acceptable adjuvants, excipients or diluents in addition to the carrier.

As used herein, the term "pharmaceutically acceptable" refers to a composition that is physiologically acceptable and does not normally cause gastrointestinal disturbances, allergic reactions such as dizziness, or similar reactions when administered to humans. Examples of such carriers, excipients, and diluents include lactose, dextrose, sucrose, sorbitol, mannitol, xylitol, erythritol, maltitol, starch, acacia gum, alginate, gelatin, calcium phosphate, calcium silicate, cellulose, methyl cellulose, polyvinylpyrrolidone, water, methylhydroxybenzoate, propylhydroxybenzoate, talc, magnesium stearate, and mineral oil. It can further include fillers, anti-flocculants, lubricants, wetting agents, flavors, emulsifiers, and preservatives.

Furthermore, the pharmaceutical composition according to one embodiment of the present invention can be formulated using methods known in the art to allow for rapid release, or sustained or delayed release of the active ingredient upon administration to a mammal. Formulations include powders, granules, tablets, emulsions, syrups, aerosols, soft or hard gelatin capsules, sterile injectable solutions, and sterile powder forms.

The composition according to one embodiment of the present invention can be administered by various routes, but preferably by intrathecal injection, intracranial injection, or intracerebroventricular injection (ICV) via the Ommaya reservoir. Given the somewhat invasive nature of the administration method, automated administration at predetermined intervals is also possible via a dosing device comprising a reservoir and pump suitably connected to a catheter inserted at the point of administration. Optionally, instead of an automated dosing device, the dosage may be administered manually at suitable intervals, such as once daily or several times daily, once weekly, twice weekly, monthly, etc.

The composition according to one embodiment of the present invention can be formulated in any suitable form with a commonly used pharmaceutically acceptable carrier. Pharmaceutically acceptable carriers include, for example, water, suitable oils, saline, carriers for parenteral administration such as aqueous glucose and glycols, and the like, and may further include stabilizers and preservatives. Suitable stabilizers include antioxidants such as sodium hydrogen sulfite, sodium sulfite, or ascorbic acid. Suitable preservatives include benzalkonium chloride, methyl- or propyl-paraben, and chlorobutanol. The composition according to the present invention may also contain suspending agents, solubilizing agents, stabilizing agents, isotonic agents, preservatives, anti-adsorbents, surfactants, diluents, excipients, pH adjusters, currency-free agents, buffering agents, antioxidants, and the like, as appropriate, depending on the method of administration or formulation. Pharmaceutically acceptable carriers and formulations suitable for the present invention, including those exemplified above, are described in detail in the literature [Remington's Pharmaceutical Sciences, latest edition].

The dosage of a composition to a patient depends on many factors, including the patient's height, body surface area, age, the specific compound being administered, gender, time and route of administration, general health, and other medications being administered concurrently. The pharmaceutically active antisense oligonucleotide may be administered in an amount of 100 ng/kg body weight (kg) to 10 mg/kg body weight (kg), more preferably in an amount of 1 to 500 µg/kg body weight (kg), and most preferably in an amount of 5 to 50 µg/kg body weight (kg), but the dosage being adjusted to take into account the above factors.

As used herein, the term "therapeutically effective amount" means an amount sufficient to treat a condition with a reasonable benefit/risk ratio applicable to medical treatment, and level of effective dose may be determined based on factors including the type and severity of the individual, age, sex, activity of the drug, sensitivity to the drug, time of administration, route of administration and rate of elimination, duration of treatment, concomitant medications, and other factors well known in the medical field. Therapeutically effective amounts of the composition of the present invention may be from 0.5 µg/kg to 1 g/kg, more preferably from 10 µg/kg to 500 mg/kg, but the effective dosage may be appropriately adjusted according to the age, sex and condition of the patient.

As used herein, the term "treatment" refers to the systematic process and activities designed to treat, cure, or alleviate any disease, disorder, or problem. While the ultimate goal is to eliminate the etiology of a disease or restore a condition to its pre-onset state, symptomatic relief that eliminates or alleviates the symptoms of a disease is also included in the scope of treatment, even if it does not eliminate the etiology of a disease or restore a condition to its pre-onset state. Furthermore, the preventive act of administering a drug according to one embodiment of the present invention before the onset of symptoms so that the symptoms do not appear or, if they do appear, are mitigated so that the patient's daily life is not significantly impaired is also included in the category of treatment. Thus, the pharmaceutical composition according to one embodiment of the invention can be used to prevent disease, and methods of treatment according to one embodiment of the invention include preventing symptoms of disease.

### MODE FOR INVENTION

The present invention will be described in more detail below by examples and experimental example. However, the present invention is not limited to the examples and experimental examples disclosed below, but may be implemented in many different embodiments, and the following examples and experimental examples are provided to make the disclosure of the invention complete and to inform completely the scope of the invention to a skilled in the art.

### Example: Design of Cacna1g Targeting Antisense Oligonucleotides

The present inventors designed 20 candidate antisense oligonucleotides (ASOs) targeting cacna1g, the gene encoding the α1G subunit (Cav3.1) among three isoforms of the T-type calcium channel (FIG. 1 and Table 1), with consideration for target specificity and stability. In addition, the present inventors used gammers comprising an internal 10 nt of DNA sequence and 5 nt of RNA sequence each with a 2'-MOE modified skeleton at the 5'-end and 3'-end, respectively, which are known to be stable and effective in the art as antisense oligonucleotide structures. In the DNA portion of the gapmer, phosphorothioate bonds (PS bonds) were used instead of conventional phosphodiester bonds (PO bonds) as sugar-to-sugar bonds, and in the RNA portion, PS bonds were used only at both ends of the last sequence, while PO bonds were used in the remaining RNA portion (FIG. 2). In addition, all uracil (U) in the RNA portion was replaced with thymine (T), and all cytosine (C) was replaced with methylated cytosine to further increase the stability of the antisense oligonucleotide.

**Table 1: Antisense oligonucleotides according to one embodiment of the present invention**

| Examples | Name | Target | Nucleic acid sequence of ASO | Length (nt) | SEQ ID Nos (Target Sequence) |
|---|---|---|---|---|---|
| 1 | a73 | Exon 1 | tcaagtagaagaaaaccacc | 20 | 2(26) |
| 2 | a74 | Intron 1 | gtcagtaaaaacctcagcct | 20 | 3(27) |
| 3 | a75 | Intron 1 | atctccttgttgtgcctcca | 20 | 4(28) |
| 4 | a76 | Intron 1 | ccaaatgagaaaaagctgtg | 20 | 5(29) |
| 5 | a77 | Intron 1 | tgactatgagagacctgtgc | 20 | 6(30) |
| 6 | a78 | Intron 1 | ctgtgtgatgttcttctgtt | 20 | 7(31) |
| 7 | a79 | Exon 6 | catagccaatgttgtcaaag | 20 | 8(32) |
| 8 | a80 | Intron 7 | ctagtgtgattccagagagc | 20 | 9(33) |
| 9 | a81 | Exon 10-Intron 10 | tacctgatgaccacaatgac | 20 | 10(34) |
| 10 | a82 | Intron 14 | tacttgattgctacttttga | 20 | 11(35) |
| 11 | a83 | Exon 6 | tggttccagttgacacaggt | 20 | 12(36) |
| 12 | a84 | Exon 8 | ccattggcatccctgtcctg | 20 | 13(37) |
| 13 | a85 | Exon 14 | tacagcttaactgtccactc | 20 | 14(38) |
| 14 | a86 | Intron 14 | ttcttagcaggtcagtaggt | 20 | 15(39) |
| 15 | a87 | Exon 20 | accttcactgtcatttcagc | 20 | 16(40) |
| 16 | a88 | Intron 26 | ggagattggaaattggacat | 20 | 17(41) |
| 17 | a89 | Exon 33 | gtgttgtagcaggtggactc | 20 | 18(42) |
| 18 | a90 | Exon 35 | tcatcaggcaaagagtcatc | 20 | 19(43) |
| 19 | a91 | Intron 37-Exon 38 | gtcagtccttattgctgcct | 20 | 20(44) |
| 20 | a92 | Exon 38 | tattgctttctcccaacagc | 20 | 21(45) |
| Crtl 1 | sh3.1_ 20mer | Exon 9 | acttgctgtccacaatcttt | 20 | 22(46) |
| Ctrl 2 | AS-CAv3.1_ 20mer | Exon 8 | cgggacccattggcatccct | 20 | 23(47) |
| Negative Ctrl 1 | mock | - | Distilled Water | | |
| Negative Ctrl 2 | scrambled | - | cctataggactatccaggaa | 20 | 24(48) |
| Negative Ctrl 3 | HTTRX | HTT gene | ctcagtaacattgacaccac | 20 | 25(49) |

### Experimental Example 1: Primary Screening

The present inventors designed antisense oligonucleotide candidates targeting cacnalg mRNA as described in Table 1 above and commissioned Mycrosynth (Germany) to prepare them. The above antisense oligonucleotides were transfected into A549 cells at a concentration of 200 nM using lipofectamine reagent, and 48 hours after transfection, the cells were lysed with lysis and RT kit (TOYOBO, Japan) and reverse transcription reaction was performed. Then, qRT-PCR was performed using Taqman probe and qRT-PCR premix (TOYOBO, Japan) to measure cacna1g gene expression levels. The probe and primer information for specific qRT-PCR is listed in Table 1 below. As a result, eight antisense oligonucleotides, including A80, A82, A91, and A92, which repeatedly knocked down gene expression by more than 50%, were selected, as shown in FIG. 3.

### Experimental Example 2: Secondary Screening

The present inventors then investigated whether the eight antisense oligonucleotides selected in the Experimental Example 1 could be taken up by cells and knockdown of the cacna1g gene at two concentrations (100 nM and 400 nM) using lipofectamine reagent.

As shown in FIG. 4, all eight antisense oligonucleotides exhibited very good knockdown efficiency at a concentration of 400 nM. However, for a80, a82, and a84, the knockdown efficiency was lower than other antisense oligonucleotides at a concentration of 100 nM.

### Experimental Example 3: Toxicity assay and in vivo gene expression regulation assay

Based on the results of cellular experiments in the Experimental Examples 1 and 2, the present inventors performed animal experiments to verify the stability and pharmacological effects of the eight antisense oligonucleotide candidates according to one embodiment of the present invention.

Specifically, the present inventors divided PO-week-old athymic C57BL/6J wild-type mice into 4-6 groups of 4-6 mice per group and administered a total of 4 µl (25 µg) of each antisense oligonucleotide (1 mM) by intracerebroventricular (ICV) injection, 2 µl in each hemisphere of the brain, The survival rate was measured after 7 days, and the brain tissues of the surviving mice were harvested, and the expression level of the cacna1g gene in the brain tissues was measured using qRT-PCR as described in the Experimental Example (FIG. 5a).

As a result, as shown in FIG. 5b, all mice treated with A88 died within 6 hours, A86 killed 4 out of 6 mice, A82 killed 2 out of 5 mice, and A84 killed 2 out of 6 mice, indicating significant toxicity. On the other hand, A89, A91, and 92 were confirmed to be relatively safe antisense oligonucleotide candidates, with only 1 out of 6 animals dying or none dying at all. On the other hand, AS-Cav3.1_20mer, a positive control used to knock down Cav3.1 in the prior art (Bourinet et al., EMBO J. 24: 315-324, 2005), was used as a positive control only in the initial screening experiment and excluded from further *in vivo* screening because it is sequence complementary only in mouse and rat, not in primate genes including monkey and human, and has a high probability of cross-reacting with other genes.

Furthermore, as a result of confirming the gene expression inhibition effect in the *in vivo* experiments, it was found that A82 and A84 showed a lower gene expression inhibition effect of CACNA1G under *in vivo* conditions than in cellular experiments, while A86, A89, A91, and A92 showed a higher gene expression inhibition effect, as shown in FIG. 5c.

Therefore, based on the results of FIGs. 5b and 5c, the present inventors selected A91 and A92 as the final candidates.

### Experimental Example 4: Cross-reaction assay

There are three isoforms of the alpha subunit of T-type calcium channels, and the homology between them is very high. In particular, the alpha subunits of these three isotypes are expressed in different organs and tissues, and non-discriminatory blockers of these isotypes are known to cause various side effects when administered systemically by blocking T-type calcium channels in non-target organs. Because of this cross-reactivity, T-type calcium channel blockers developed to date have been associated with serious side effects in clinical use.

Therefore, the development of antisense oligonucleotides that can selectively inhibit the expression of only the alpha subunit (Cav3.1) without inhibiting the expression of other isoforms is a prerequisite for the development of safer drugs.

Accordingly, the inventors investigated whether a91 and a92, which were previously screened as final candidates, selectively inhibit Cav3.1 alone in animal models.

Specifically, as a control, the inventors administered a total of 4 µl (25 µg) of a91 and a92 (1 mM) to PO-week-old athymic C57BL/6J wild-type mice by intracerebroventricular (ICV) injection, 2 µl in each hemisphere of the brain, and then harvested brain tissue from the mice 7 days later to determine the expression of cacnalg (Cav3. 1), cacna1h (Cav3.2) and cacna1i (Cav3.3) genes were measured by qRT-PCR using the primers and probes listed in Table 1 and described in Experimental Example 1 above (FIG. 5a).

**Table 2: Information of primers and probes used in qPCR**

| Target Gene | Name | Nucleotide Sequence (5' -> 3') | SEQ ID NOs |
|---|---|---|---|
| Cav3.1 | Cav3.1 probe | tgtcattgggcagagagtgtgtcc | 50 |
| | Cav3.1 qPCR-F | gaggaagtctggtgtcagcc | 51 |
| | Cav3.1 qPCR-R | tgtcctagggatctctcggc | 52 |
| Cav3.2 | Cav3.2 probe | accttggtcttcttttcatgctcctgt | 53 |
| | Cav3.2 qPCR-F | gacactgtggttcaagctct | 54 |
| | Cav3.2 qPCR-R | ttatcctcgctgcattctagc | 55 |
| Cav3.3 | Cav3.3 probe | acactgtgtcctcagagcaagcag | 56 |
| | Cav3.3 qPCR-F | gggagggatgttcaggcatc | 57 |
| | Cav3.3 qPCR-R | gagcttccttcatccccacc | 58 |

As a result, it was determined that the antisense oligonucleotides A91 and A92 according to one embodiment of the present invention specifically inhibit the expression of CACNA1G, while not inhibiting the expression of genes encoding other isoforms, as shown in FIGs. 6a and 6b. Thus, it can be seen that antisense oligonucleotides according to one embodiment of the present invention can be efficiently used for the treatment of various diseases in which the alpha 1G subunit is involved by selectively inhibiting the expression of only the alpha 1G subunit without affecting the expression of other isoforms.

### Example 5: Analyzing the therapeutic effect on Parkinson's disease

From the results of the Experimental Examples 1 to 4, the present inventors investigated whether the antisense oligonucleotide according to one embodiment of the present invention would exhibit therapeutic effects on Parkinson's disease, which is known to be a representative disease among CACANA1G-related diseases.

### 5-1: Verification of therapeutic effect

Specifically, the present inventors used mice treated with 6-hydroxydopamine (6-OHDA) as a model animal for Parkinson's disease, and then administered 200 µg of the antisense oligonucleotide (a91) according to one embodiment of the present invention to 7-8 week old athymic C57BL/6J wild-type mice via intracerebroventricular injection and performed an open field assay one week later (FIG. 7a).

As a result, as shown in FIG. 7b, open field analysis 1 week after injection of antisense oligonucleotides according to one embodiment of the present invention revealed reduced ipsilateral rotation upon administration of antisense oligonucleotide (A91) according to one embodiment of the present invention.

A Parkinson's disease model mouse induced by unilateral injection of 6-OHDA into the striatum characterized by a phenotype of repetitive rotation in the direction of injection (Thiele et al., J. Vis. Exp., 60: e3234, 2012). In the case of the mice used in FIGs. 7b and 7c, 6-OHDA was injected into the striatum located in the left hemisphere, resulting in a phenotype of leftward rotation. This phenotype of rotating in the same direction as the injection is known as ipsilateral rotation. In Figure 7b, the ratio of coaxial rotation to distance traveled is increased in the group of mice injected with 6-OHDA alone, while the ratio of ipsilateral rotation is decreased in mice injected with intracerebroventricular injection of 200 µg of Cav3.1-ASO (a91) in addition to the 6-OHDA model. This suggests that the injection of 200 µg of Cav3.1-ASO reduces the 6-OHDA-induced pathological phenotype of Parkinson's disease.

In FIG 7c, the distance traveled by the mice was measured over a 30-minute period. Although the mice treated with an additional 200 µg of Cav3.1-ASO may appear to have reduced movement to the level of the negative control, there was no statistically significant difference in distance traveled in any of the groups. Furthermore, FIG. 7d shows the movement speed of these mice. Similar to the distance traveled above, the mice treated with an additional 200 µg of Cav3.1-ASO showed a decrease in locomotion to the level of the negative control, but there was no statistically significant difference in locomotion in any of the groups.

The present inventors then performed an accelerated rotarod test as an endpoint for locomotor performance. The accelerated rotarod test was performed on the above animals 1 week after antisense oligonucleotide injection and at 1-day intervals.

As a result, as shown in FIG. 7e, the antisense oligonucleotide according to one embodiment of the present invention improved exercise performance in a dose-dependent manner and with increasing test days. In contrast, in the negative control group treated with 6-OHDA alone, the ataxia did not improve over time.

### 5-2: Identification of Mechanism

The present inventors determined whether the mechanism of action of antisense oligonucleotides according to one embodiment of the present invention actually operated after the death of dopamine neurons.

To do so, the inventors specifically administered the drug 6-OHDA, a known Parkinson's disease-inducing drug, to the striatum of the brain in the experimental animals of the Example 5-1, and co-administered the antisense oligonucleotide on the same day of administration. Since 6-OHDA induces cell death within a week and the antisense oligonucleotide takes about a week to work, it is difficult to expect an inhibitory effect on death of dopamine neurons using this method. To measure dopaminergic neuronal death, the animals were sacrificed after 5 weeks, the brains were excised, tissue sections were prepared using a cryotome, and histochemical analysis was performed on the striatum and substantia nigra using an antibody that specifically binds to tyrosine hydroxylase (TH), a marker specific for dopaminergic neurons. As shown in FIG. 7f, dopaminergic neurons in the control group without any drug appeared normal, but death of dopaminergic neurons was observed in all groups treated with 6-OHDA. This shows that the antisense oligonucleotide did not affect dopaminergic neuron death in the experimental method used in this embodiment, but works by blocking the abnormal motor neuron signaling that occurs after dopaminergic neurons die, which follows the loss of dopaminergic neurons.

### Example 6: Analysis of treatment effectiveness in essential tremor

The inventors next investigated whether an antisense oligonucleotide according to one embodiment of the present invention is effective in the treatment of essential tremor by performing animal experiments on harmaline-induced essential tremor model mice.

To this end, specifically, as shown in FIG. 8a, 200 µg of antisense oligonucleotide (a91) according to one embodiment of the present invention was administered intracerebroventricularly to 7-8 week old athymic C57BL/6J wild-type mice, followed 2 weeks later by intraperitoneal administration of harmaline, a tremor inducer, at a dose of 9 mg/kg, and 8-10 minutes later, a tremor test was performed using the same apparatus as shown in FIG. 8b.

As a result, as shown in FIGs. 8c and 8d, the antisense oligonucleotide according to one embodiment of the present invention significantly inhibited tremor compared to the control (wild type) or vehicle (control ASO treatment).

The above results demonstrate that the antisense oligonucleotide according to one embodiment of the present invention is effective in the treatment of essential tremor.

### Example 7: Analysis of the effectiveness in treating depression

**The inventors next investigated whether the antisense oligonucleotide** according to one embodiment of the present invention is effective in the treatment of ET by performing animal experiments on mice in a stress-induced depression model.

### 7-1: Effectiveness in a chronic social stress model

First, the present inventors investigated the depression scale using the experimental schedule shown in FIG. 9a and the specific method shown in FIG. 9b, Specifically, 200 µg of the antisense oligonucleotide (a91) according to one embodiment of the present invention was injected intracerebroventricularly into 7-8 week old athymic C57BL/6J wild-type mice, followed by social defeat stress from day 5 to day 10 by merging another aggressive individual into the cage in which the animals were housed, and then the degree of stress-induced depression was measured by measuring social interactions on day 17.

As a result, as shown in FIG. 9c, experimental animals treated with an antisense oligonucleotide according to one embodiment of the present invention had reduced Cav3.1 expression, while social interaction was restored to almost control levels. This indicates that the antisense oligonucleotide according to one embodiment of the present invention is a highly effective agent for the reduction of social stress.

### 7-2: Effectiveness in a short-term acute stress model

Next, the present inventors performed a forced swimming test to determine whether the antisense oligonucleotide according to one embodiment of the present invention has a therapeutic effect on acute depression.

Specifically, 200 µg of antisense oligonucleotide (a91) according to one embodiment of the present invention was administered intracerebroventricularly to 7-8 week old athymic C57BL/6J wild-type mice and after 3 weeks, the mice were placed in a water bath as shown in FIG. 10a and the stress index was determined by measuring the immobility time caused by stress.

The results confirmed that the antisense oligonucleotide according to one embodiment of the present invention not only significantly lowered the expression of Cav3.1 in the brain, as shown in FIG. 10b, but also significantly reduced the immobility time, which is a sign of stress. These results demonstrate that the antisense oligonucleotide according to one embodiment of the invention is a substance that confers tolerance to short-term stress as well as chronic stress.

### Example 8: Effectiveness in treating epilepsy

The present inventors investigated whether an antisense oligonucleotide according to one embodiment of the present invention can be used to treat epilepsy.

Specifically, as shown in FIG. 11a, the inventors administered 200 µg of an antisense oligonucleotide (a91) according to one embodiment of the present invention intracerebroventricularly to 7-8 week old athymic C57BL/6J wild-type mice, followed by an EEG probe insertion procedure at 12 days and an absence seizure-inducing gamma-butyrolactone (GBL) at 70 mg/kg at 14 days. The electroencephalogram (EEG) was measured 30 minutes before and after GBL administration, and at 42 days after antisense oligonucleotide administration, the animals were sacrificed and the brains were harvested to measure Cav3.1 mRNA expression levels.

As a result, as shown in FIG. 11b, the antisense oligonucleotide according to one embodiment of the present invention significantly reduced both the frequency and the number of spike and wave discharges (SWDs) per 10 minutes.

These results demonstrate that antisense oligonucleotides according to one embodiment of the present invention are effective in the treatment of epilepsy, particularly absence seizures.

### Example 9: Restoration of consciousness

Coma is a medical term for a state of deep unconsciousness. A comatose patient, also known as a "vegetative patient" cannot be awakened and does not respond to external stimuli such as pain, light, or sound. There are many known causes of coma, including drug intoxication, abnormal metabolism, central nervous system disease, hypoxia, and stroke, but coma can also be caused by trauma to the brain, such as a car accident or fall. Unfortunately, no drug has been reported to restore consciousness to such comatose patients.

Loss of consciousness is a major symptom of epilepsy as discussed in the Example 8. Furthermore, given the well-known correlation of epilepsy with T-type calcium channels and seizures, and the results of the Example 8 demonstrating that inhibition of the expression of the alpha1G subunit of T-type calcium channels can alleviate the symptoms of seizures, the present inventors sought to determine whether antisense oligonucleotides according to one embodiment of the present invention are effective in restoring consciousness.

To this end, the present inventors specifically administered 200 µg of antisense oligonucleotide (a91) according to one embodiment of the present invention intracerebroventricularly to 7-8 week old athymic C57BL/6J wild-type mice, as shown in FIG. 12a, and induced loss of righting reflex (LORR) 11 days later by intraperitoneal administration of an anesthetic, Avertin 400 mg/kg. The loss of righting reflex (LORR) duration was then measured. As a result, as shown in FIG. 12b, LORR duration was significantly reduced in the antisense oligonucleotide (A91) treatment group according to one embodiment of the present invention.

The present inventors further investigated whether an antisense oligonucleotide according to one embodiment of the present invention could rapidly restore alcohol-induced loss of consciousness using an alcohol-induced loss of consciousness model animal.

Specifically, 200 µg of an antisense oligonucleotide (a91) according to one embodiment of the present invention was administered intracerebroventricularly to 7-8 week old athymic C57BL/6J wild-type mice according to the experimental schedule shown in FIG. 12C, and after 12-13 days, the mice were treated with a 20% aqueous solution of ethanol at a dose of 0. 6 g/kg intraperitoneally at a dose of 0. 6 g/kg, the behavior of the animals was videotaped in the open field for 30 min, and the movement speed, distance traveled, and frequency of turns were measured using computer software (EthoVision, Noldus, Netherland). After two days, loss of consciousness was induced by intraperitoneal administration of a high concentration (3.5 g/kg) of ethanol and the latency to loss of consciousness (LORR latency) was measured. Furthermore, 21 days after administration of oligonucleotide (A91) according to one embodiment of the present invention, the animals were sacrificed and their brains were harvested to measure Cav3.1 gene expression.

As a result, as shown in FIG. 12d, more vigorous movements were observed in the antisense oligonucleotide treatment group according to one embodiment of the present invention, and in the case of loss of consciousness latency, the antisense oligonucleotide treatment group according to one embodiment of the present invention showed a longer time compared to the control group.

This indicates that the antisense oligonucleotide according to one embodiment of the present invention can promote the recovery of consciousness in patients in a state of loss of consciousness by specifically inhibiting the expression of the Cav3.1 gene, and shows that the antisense oligonucleotide of the present invention is a promising substance that can be developed as an inducer of recovery of consciousness in comatose patients for which no suitable treatment exists.

### Examples 21-23: ASO Target Site Identification

From the results of the above examples, the present inventors sought to more precisely identify the target site of the antisense oligonucleotide according to one embodiment of the present invention.

For this purpose, a plurality of target sequences (A4, A7, and A11) selected within SEQ ID NO: 1 and located in the vicinity of the antisense oligonucleotide (A91) according to one embodiment of the present invention, taking into account the location of the CACNA1G genomic gene as shown in Table 4, were determined, and their safety and inhibition of Cav3.1 expression were investigated.

**Table 3: Sequences surrounding the antisense oligonucleotide targeting SEQ ID NO: 1.**

| Name (Examples) | Location on genome | | Genomic Sequence (SEQ ID Nos) | ASO Sequences (SEQ ID NOs) |
|---|---|---|---|---|
| | 5' | 3' | 5'->3' | 5'->3' |
| a91(19) | 65301 | 65320 | aggcagcaataaggactgac(44) | gtcagtccttattgctgcct(20) |
| a4(21) | 65553 | 65572 | tggacacggagctgagctgg(59) | ccagctcagctccgtgtcca(62) |
| a7(22) | 66293 | 66312 | gaaagcaatacgtttgtgca(60) | tgcacaaacgtattgctttc(63) |
| a11(23) | 66733 | 66392 | gacattttgtgactgaagag(61) | ctcttcagtcacaaaatgtc(64) |

The genomic locations in Table 3 are based on the genomic nucleic acid sequence of the cacnalg gene as described in GenBank Reference Sequence NC_000017.11 REGION: 50560715..50627474.

Specifically, the present inventors divided PO-week-old C57BL/6J wild-type mice into four to six groups per group and administered a total of 4 µl (25 µg) of the antisense oligonucleotide (1 mM) listed in Table 3 by intracerebroventricular (ICV) injection, 2 µl in each hemisphere of the brain, The survival rate was measured after 7 days, and the brain tissues of the surviving mice were harvested, and the expression level of the cacna1g gene in the brain tissues was measured using qRT-PCR as described in the Example 1 (FIG. 13a).

As a result, as shown in FIG. 13b, the newly designed antisense oligonucleotides of the present invention exhibited similar or superior survival rates compared to the control group and the antisense oligonucleotide (A91) according to one embodiment of the present invention, except for A4, indicating high safety.

Furthermore, the effect on Cav3.1 expression was examined, and as shown in FIG. 13c, among the newly designed antisense oligonucleotides, all showed a significant, although somewhat weaker, inhibition of Cav3.1 mRNA expression than a91, and a4 and a7 also showed inhibition of Cav3.1 mRNA expression, although not statistically significant. The cause of the statistical insignificance is that one individual in each experimental group showed a higher gene expression rate than the control group, which is judged to be due to experimental error, and it is judged that increasing the number of experimental heads will show a statistically significant decrease in gene expression.

As can be seen from the above results, the location on the cacna1g genomic gene selected in the present invention is, contrary to expectation, close to the 3'-terminus of the pre-spliced mRNA, and it can be seen that any site within SEQ ID NO 1, including intron 37 and exon 38, can efficiently reduce the expression of Cav3.1 mRNA and be useful for the treatment of neurological diseases associated with alpha 1G T-type calcium channels.

As described above, the antisense oligonucleotides according to one embodiment of the present invention not only selectively inhibit the expression of the alpha 1G subunit without affecting the expression of other isoforms of T-type calcium channels, but also show remarkable effectiveness in the treatment of diseases associated with the alpha 1G subunit, in particular Parkinson's disease, without any other side effects. Thus, antisense oligonucleotides according to one embodiment of the present invention can be used as therapeutic agents for the treatment of various diseases related to the alpha 1G subunit of T-type calcium channels, including Parkinson's disease, as well as various neurodegenerative diseases such as depression, essential tremor, epilepsy, anxiety disorders, and unconsciousness.

The invention has been described with reference to the above-described embodiments and experimental examples, but these are exemplary only, and one of ordinary skill in the art will understand that various modifications and other equally valid embodiments are possible therefrom. Therefore the true scope of the invention should therefore be determined by the technical features of the appended claims.

### INDUSTRIAL APPLICABILITY

Antisense oligonucleotides according to one embodiment of the present invention selectively inhibit the expression of alpha 1G T-type calcium channels and can be used as medicines to treat neuropsychiatric disorders such as Parkinson's disease, depression, essential tremor, epilepsy, and loss of consciousness, as well as to restore consciousness in unconscious patients.

## Claims

1. An antisense oligonucleotide targeting the nucleic acid sequence represented by SEQ ID NO: 1 for inhibiting the expression of a gene encoding Cav3.12.

2. The antisense oligonucleotide of claim 1, wherein the antisense oligonucleotide is capable of hybridizing to the nucleic acid sequence of SEQ IOD NO: 1 and has a length of 11 to 30 nt.

3. The antisense oligonucleotide of claim 1, wherein the antisense oligonucleotide is DNA, RNA, or DNA/RNA chimeric nucleic acid molecule.

4. The antisense oligonucleotide of claim 1, wherein the antisense oligonucleotide comprises nucleic acid sequence of SEQ ID NO: 20 or SEQ ID NO: 21.

5. The antisense oligonucleotide of claim 1, wherein some or all of the phosphodiester (PO) backbone is substituted with phosphorothioate (PS), phosphorodithioate, or methylphosphonate.

6. The antisense oligonucleotide of claim 1, wherein some or all of the 2'-OH of the sugar portion is modified.

7. The antisense oligonucleotide of claim 6, wherein the 2'-OH of the sugar portion is substituted with -O-CH₃ (-OMe), -O-CH₂-CH₃ (-OEt), -O-CH₂-CH₂-O-CH₃ (-MOE), -O-CH₂-CH₂-CH₂-NH₂, -O-CH₂-CH₂-CH₂-OH, -OF or -F.

8. The antisense oligonucleotide of claim 1, wherein the sugar portion comprises at least one nucleotide analog having an additional ring formed thereon.

9. The antisense oligonucleotide of claim 8, wherein the nucleotide analog is a locked nucleic acid (LNA).

10. The nucleotide analog of claim 8, wherein the nucleotide analog is at least 33% of the total nucleotides.

11. The antisense oligonucleotide of claim 1, wherein the antisense oligonucleotide that is a gapmer.

12. The antisense oligonucleotide of claim 11, wherein said gapmer is a nucleic acid molecule comprising an RNA unit of 3-7 nt in length at each end and DNA of 8-12 nt in length spaced between said RNA units.

13. The antisense oligonucleotide of claim 12, wherein some or all of said RNA is LNA.

14. A pharmaceutical composition for treating Parkinson's disease, comprising the antisense oligonucleotide of any one of claims 1 to 13.

15. A pharmaceutical composition for treating depression, comprising the antisense oligonucleotide of any one of claims 1 to 13.

16. A pharmaceutical composition for treating essential tremor, comprising the antisense oligonucleotide of any one of claims 1 to 13.

17. A pharmaceutical composition for treating epilepsy, comprising the antisense oligonucleotide of any one of claims 1 to 13.

18. A pharmaceutical composition for treating an anxiety disorder, comprising the antisense oligonucleotide of any one of claims 1 to 13.

19. A pharmaceutical composition for recovering consciousness in a subject in an unconscious state, comprising the antisense oligonucleotide of any one of claims 1 to 13.

20. A method of treating a subject suffering from Parkinson's disease, comprising administering a therapeutically effective amount of an antisense oligonucleotide of any one of claims 1 to 13 to the subject.

21. A method of treating a subject suffering from essential tremor, comprising administering a therapeutically effective amount of an antisense oligonucleotide of any one of claims 1 to 13 to the subject.

22. A method of treating a subject suffering from depression, comprising administering a therapeutically effective amount of an antisense oligonucleotide of any one of claims 1 to 13 to the subject.

23. A method of treating a subject suffering from epilepsy, comprising administering a therapeutically effective amount of an antisense oligonucleotide of any one of claims 1 to 13 to the subject.

24. A method of treating a subject suffering from an anxiety disorder, comprising administering a therapeutically effective amount of an antisense oligonucleotide of any one of claims 1 to 13 to the subject.

25. A method of recovering consciousness in a subject in an unconscious state comprising administering a therapeutically effective amount of an antisense oligonucleotide of any one of claims 1 to 13 to the subject.
